# EUROPEAN PATENT APPLICATION

(11) **EP 1 712 131 A2**
(43) Date of publication of application: **18.10.2006**
(21) Application number: 06380074.2
(22) Date of filing: 10.04.2006
(51) Int. Cl.: A01N 43/38, A01N 25/00

(54) **Formulations comprising indole and/or indole derivatives for increasing the capacity of plants to assimilate mineral nutrients.**

(30) Priority: 11.04.2005 ES 200500841
(71) Applicant: INABONOS, S.A., 31160 Orcoyen (Navarra) (ES)
(72) Inventor: Garcia-Mina Freire, José Mariá, 31170 IZA (Navarra) (ES); Bacaicoa, Eva, 31007 Pamplona (Navarra) (ES); San Francisco Elia, Sara, 31010 Barañain (ES); Aguirre Rodriguez, Elena, 31600 Burlada (Navarra) (ES); Lemenager, Diane, 31150 Mendigoria (Navarra) (ES); Zamarreño Arregui, Ángel María, 31638 Eugui (Navarra) (ES)
(74) Representative: Del Santo Abril, Natividad

(57) **Abstract**

The object of the present invention is a new set of formulations with the potential to increase the efficiency of a plant's assimilation of nutrients both in normal conditions and in conditions of nutrient deficiency. It is especially effective in the case of iron assimilation in conditions of deficiency of this micronutrient, by improving and potentiating a plant's specific response to iron deficiency, increasing the plants capacity to mobilize and assimilate the iron present in non-assimilable forms.

## Description

The object of the present invention is a new set of formulations with the potential to increase the efficiency of plants to assimilate different mineral nutrients both in normal nutritional conditions and also when there are deficiencies of this micronutrient, especially in the case of iron assimilation in conditions of deficiency of this micronutrient, by improving and enhancing the specific response of plants to iron deficiency, increasing the plant's ability to mobilize and assimilate iron present in soils in a non-assimilable form for plants.

### BACKGROUND OF THE INVENTION

One of the most important problems affecting crops growing in basic and limestone soils is the deficiency of some nutrients due to the formation in the soils of salts of these nutrients with some soil components that are barely soluble in water. These nutrients include phosphorus, metal nutrients (iron, copper, magnesium and zinc) and boron. Of these nutrients, the one that causes the most problems for crop development is iron (Sanz, M, Cavero, J Abadia, J 1992), iron chloroels in the Ebro river basin, Spain. J.Plant. Nutr. 15, 1971-1981). In fact, although iron is one of the most abundant elements in nature and also in agricultural soils, in conditions of basic pH such as limestone soils-the majority soil in many Mediterranean areas-the assimilable concentration of iron is greatly reduced due to the formation of largely insoluble compounds (hydroxides, oxides, phosphates, carbonates etc). This causes the crops growing in these soils to present severe growth problems associated with iron deficiency, depending on the efficacy of the varieties to uptake the iron present in the soils, if iron is not added in an assimilable form (Sanz, M., Cavero, J, Abadia, J, 1992. Iron chlorosis in the Ebro river basin, Spain, JPlant Nutr. 15, 1971-1981).

The problem of plant assimilation of the above nutrients, especially iron, in adverse soil conditions is mainly due to two factors:
1. The presence of fractions of these nutrients potentially assimilable for the plants in the soil solution in the rhizosphere.
2. The plant's potential to mobilize and assimilate these nutrients, especially iron.

Indeed, different studies have shown that plants have the ability to activate a series of specific mechanisms to improve their efficiency in iron assimilation in conditions of deficiencies of this micronutrient. These mechanisms depend on the type of plant (Schmidt, W., 1999. Mechanisms and regulation of reduction-based iron uptake in plants Review New Phytol. 141, 1-26) Hence, dicotyledon and monocotyledon non-graminaceous plants activate a set of responses, called Strategy I, that include acidification of the rhizosphere, the release of organic compounds with a reducing and complexing activity, activation of the enzyme called chelate-reductase that permits the reduction of iron (III) to iron (II), an increase in the concentration of specific transporters of Fe (II) to the interior of the root, and the generation of specific morphological changes in the root (proliferation of lateral roots and absorbent hairs, apical thickening etc.). However, graminaceous plants activate a response, called Strategy II, that mainly consists in the release of specific organic compounds with the ability to complex and solubilize iron in the rhizosphere. These compounds are called phytosiderophores

Undoubtedly, all the products or global strategies designed to resolve the problem of the deficiency of these nutrients, and especially iron, in plants, must affect at least one of the aforementioned aspects.

Currently, research in this area has focused on the development of products containing the different nutrients which can maintain these nutrients in an assimilable form in basic and limestone soils.

In the case of iron, the best developed products have been defined as organic chelates, among which EDDHA chelates and EDDHA analogues are noteworthy (for example: Knell, M Rroll, H. US2921847, 1960; Pertee, H, Stutts, J. US3981712, 1976; Niggemann, J., Mues, V. ZA7901767, 1980; Lewkowycz, KR. ES2019247., 1991) although also other products containing potentially assimilable iron have also been developed (E.g. Hawkins, E, Clapp, J, Sansing, J US5019149, 1991; Bienfalt, HF, NL1 01 9563C, 2003).

There is, therefore, the need to find a product that can stimulate the response of the plant to the deficiency of the aforementioned nutrients and especially iron, improving the efficiency of these plants to mobilize and assimilate these nutrients, especially iron, from the soil components.

### DETAILED DESCRIPTION OF THE INVENTION

This new invention consists in liquid or solid formulations containing indol or some derivatives of indol according to the following general formula: 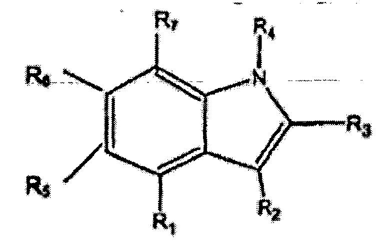 where R₁, R₂, R₃, R₄, R₅, R₆ and R₇ can be hydrogen or an alkyl, aryl or phenyl residue.
Optionally, the formulation of the invention can be accompanied by standard nutrients and/or biostimulants.
From this formulation, indol-acetic acid and its derivatives are excluded, which are compounds of specific auxinic activity, and when added exogenously to plants do not present specific activity of the object formulations of the present invention, although they do have a biostimulant effect.
The optimum doses to apply are those with a concentration of the active ingredient in the range 10⁻⁶M to 10⁻⁹ M, can present as a solid or a liquid and can be applied to the leaves or the roots.
This new compound can be composed of products containing one or several mineral nutrients such as nitrogen, phosphorus, potassium, calcium, magnesium, iron, copper, manganese, boron, titanium, nickel, molybdenum and zinc (the elements of a metallic character can be presented in the free form or as chelates with organic compounds), and/or biostimulants such as humic and fulvic acids, seaweed extracts, amino acids (such as methionine, tryptophan, glycine-betsine and glycine) or plant hormones, the most noteworthy of which correspond to cytokines, auxins, gibberellins, abscisic acid, salicylic acid, ethylene or products that release ethylene, nitric oxide or products that release nitric oxide, polyamines and brasinosteroids; or hormonal precursors such as adenine, adenosine, cyclic nucleotides or isopentyl alcohol.

The product can be applied at any time during the cycle but the most appropriate moment is in the first stages of plant development in annual plants or with the movement of sap in woody plants.
The formulation of the present invention is obtained by directly mixing the components.

### EXAMPLES

As an example, a series of specific formulations is described below that is obtained by directly mixing the different components indicated. These examples are given to illustrate more clearly the contents of the invention and do not at all limit its contents.

### Example 1

A product obtained by directly mixing the following components in the proportions indicated (% in weight):
- 0.01 % indol
- 5% ether
- 10% Ethanolamine
- 84.99% water

### Example 2.

A product obtained by directly mixing the following components in the specified proportions (% in weight):
- 0.01% Indol
- 5% ether
- 10% ethylamine
- 4% chelated Fe (E.g. Fe-EDDHA)
- 5% humic acids
- 5% seaweed extract
- 70.99% water

### Example 3.

A product obtained by directly mixing the following components in the specified proportions (% in weight):
- 1% indol
- 1% D,L, Tryptophan
- 10% N-methyl pyrrolidone
- 20% iron chloride
- 68% water

### Example 4.

A product obtained by a direct mixture of the following components in the proportions specified (% in weight):
- 1% indol
- 60% powdered potassium humate
- 39% powdered seaweed extract

### Comparative example

The present Example shows the effect of a composition containing indol on the assimilation of *iron* and the development in pepper crop plants grown in hydroponia.
Four different treatments are presented that are applied to peppers:
1. The control received a nutritive solution containing different nutrients.
2. The treatment AIA that received the same nutritive control solution was also treated with indol-acetic acid (natural auxin) at a dose of 10⁻⁶ M.
3. The treatment IND1 that received the same nutritive control solution was also treated with the composition of Example 1 at a dose of 10⁻⁶ M.
4. The treatment IND2 that received the same nutritive control solution was also treated with the composition of Example 1 at a dose of 10⁻⁹ M.

The pepper plants were cultivated in hydroponia.
The results obtained are compiled in Table 1. As can be observed, the treatments with indol corresponding to the formulation of Example 1 were associated with an important rise in iron assimilation correlated with a significant reduction in iron accumulated in the roots, indicating a better real assimilation and translocation of this element from the root to the aerial part. This effect was associated with a significant increase in plant development.
As can be demonstrated, indol-acetic acid-a natural auxin-although it presents a biostimulant effect, has no significant effects on the assimilation and translocation of iron.
Assays were repeated to measure the effect with different micronutrients and macronutrients, observing similar behaviors. The micronutrients assayed were copper, manganese, zinc and boron, and the macronutrients were phosphorus, potassium, calcium and magnesium.

**Table 1. Effects of different compositions on iron assimilation and the development of pepper plants cultivated in hydroponia.**

| Treatments | Assimilated Fe mg Fe mg plant⁻¹ (%) | Assimilated Fe mg Fe mg plant⁻¹ (%) | | Dry matter g plant⁻¹ (%) | |
|---|---|---|---|---|---|
| | leaves | root | leaves | root | stem |
| Control | 0,324 (100) | 2,64 (100) | 2,97 (100) | 1,10 (100) | 0,79 (100) |
| AIA 10⁻⁶ M | 0,335 (103,4) | 2,90 (109,8) | 4,04 (136) | 1,43 (130) | 1,09 (138) |
| IND1 10⁻⁶ M | 0,370 (114,2) | 1,56 (59) | 4,06 (136,7) | 1,45 (132) | 0,88 (111) |
| IND2 10⁻⁹ M | 0,480 (148,2) | 1,35 (51,1) | 4,97 (167,3) | 1,37 (122) | 1,19 (151) |

## Claims

1. Formulation to increase a plant's assimilation of iron in normal conditions and in conditions of deficiency of this micronutrient, but also of copper, magnesium, zinc, boron, phosphorus, potassium, calcium or magnesium; through the root or leaf, consisting in one or several of the designated compounds presenting the following formula: 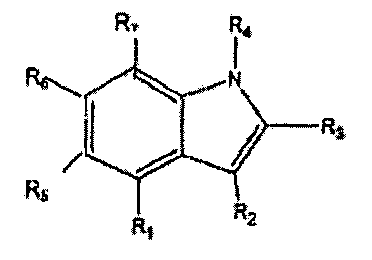 Where R₁, R₂, R₃, R₄, R₅, R₆ and R₇ can be hydrogen, an alkyl, aryl or phenyl residue.

2. Formulation according to claim 1 in which the designated compound is the indol.

3. Formulation according to claim 2 in which indol is formulated with tryptophan (D or L isomer or the racemic mixture D,L).

4. Formulation according to any of claims 1 to 3 which also includes mineral nutrients and/or biostimulants.

5. Formulation according to claim 4 in which the mineral nutrients are selected from among nitrogen, phosphorus, potassium, calcium, magnesium, iron, copper, manganese, boron, titanium, nickel, molybdenum, zinc and its mixtures.

6. Formulation according to either of claims 4 or 5 in which the biostimulants are selected from among humic and fulvic acids, seaweed extracts, methionine, glycine-betaine, tryptophan, betains, glycine, cytokines, auxins, gibberellins, abscisic acid, salicylic acid, ethylene or products that release ethylene, nitric oxide or products that release nitric oxide, polyamines, brasinosteroids, adenine, adenosine, cyclic nucleotides, alcohol isopentyl or its mixtures

7. Formulation according to any of claims 1 to 6 that can be presented as a liquid or solid formulation.

8. Formulation according to claims 1-7 that can be applied to the leaves or roots of all types of plants.
